(19) Europäisches Patentamt
European Patent Office
Office européen des brevets

(11) Veröffentlichungsnummer: **0 260 485**
**B1**

(12) # EUROPÄISCHE PATENTSCHRIFT

(45) Veröffentlichungstag der Patentschrift: **04.07.90**

(51) Int. Cl.[5]: **C07D 213/61** // C07D213/26

(21) Anmeldenummer: **87112284.2**

(22) Anmeldetag: **25.08.87**

(54) **Verfahren zur Herstellung von 2-Chlor-5-Chlormethylpyridin.**

(30) Priorität: **04.09.86 DE 3630046**

(43) Veröffentlichungstag der Anmeldung: **23.03.88 Patentblatt 88/12**

(45) Bekanntmachung des Hinweises auf die Patenterteilung: **04.07.90 Patentblatt 90/27**

(84) Benannte Vertragsstaaten: **BE CH DE FR GB IT LI NL**

(56) Entgegenhaltungen:
**US-A- 3 931 200**
**US-A- 4 205 175**

**SYNTHESIS, Nr. 8, August 1984, Seiten 676-679; G.R. NEWKOME et al.: "Alpha-methyl functionalization of electron-poor heterocycles: free radical chlorination"**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Gallenkamp, Bernd, Dr., Claudiusweg 5, D-5600 Wuppertal 1(DE)**

**EP 0 260 485 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft eine neues Verfahren zur Herstellung von 2-Chlor-5-chlormethyl-pyridin, welches beispielsweise als Zwischenprodukt für die Herstellung von Insektiziden verwendet werden kann.

Es ist bereits bekannt, daß 5-Chlormethylpyridine durch Umsetzung von 5-Hydroxymethylpyridinen mit Chlorierungsmitteln wie z.B. Thionylchlorid erhalten werden können (vgl. EP-OS 163 855 und J. Het. Chem. 16, 333 (1979)). Nachteilig bei diesem Verfahren sind die vielen Reaktionsstufen, die für die Herstellung der 5-Chlormethylpyridine erforderlich sind.

Weiterhin ist bekannt, daß eine direkte Chlorierung der Methylgruppe von 3-Methylpyridinen nicht möglich ist (vgl. Helv. Chim. Acta 59, 179 ff (1976) und Angew. Chem. 1963, 236 ff).

Es wurde nun gefunden, daß man 2-Chlor-5-chlormethyl-pyridin der Formel (I)

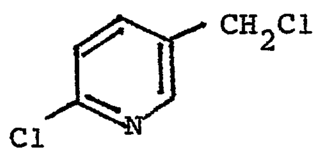

(I)

erhält, wenn man 2-Chlor-5-methylpyridin der Formel (II)

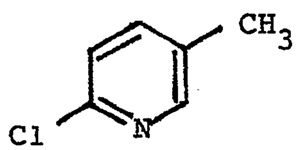

(II)

gegebenenfalls in Gegenwart von Säureakzeptoren und gegebenenfalls in Gegenwart von inerten Verdünnungsmitteln bei Temperaturen zwischen 0°C und 100°C mit elementarem Chlor chloriert.

Überraschenderweise gelingt es mit Hilfe des erfindungsgemäßen Verfahrens auf einfache Weise und mit geringem Aufwand, aus 2-Chlor-5-methylpyridin durch direkte Chlorierung das entsprechende 2-Chlor-5-chlormethylpyridin herzustellen.

Nach dem Stand der Technik sind für die Herstellung von 5-Chlormethylpyridinen 4 aufwendige Reaktionsstufen erforderlich:

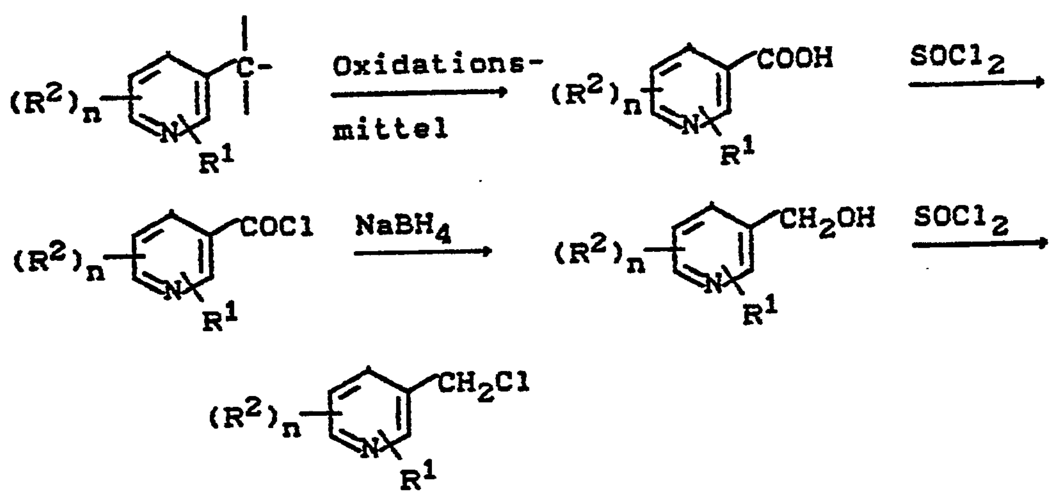


(vgl. J. Org. Chem. 34, 3545 (1969) und J. Het. Chem. 16, 333 (1979)). Diese problematische Reaktionssequenz kann nun in überraschend einfacher Weise umgangen werden.

Für das erfindungsgemäße Verfahren verwendet man 2-Chlor-5-methyl-pyridin und elementares Chlor als Ausgangsstoffe.

Die Reaktion kann durch das folgende Formelschema wiedergegeben werden:

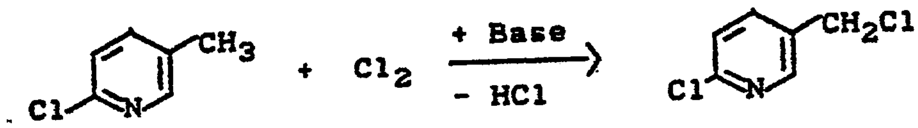


Das erfindungsgemäße Verfahren zur Herstellung der Verbindung der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische, gegebenenfalls halogenierte Kohlenwasserstoffe wie Methylenchlorid, Ethylenchlorid, Chloroform, Tetrachlorkohlenstoff, Ether wie Diethyl- und Dibutylether, Methyl-tert.-butylether, Glykoldimethylether und Diglykoldimethylether, Tetrahydrofuran und Dioxan.

Das erfindungsgemäße Verfahren wird bevorzugt in Gegenwart von Säureakzeptoren durchgeführt. Als Säureakzeptoren können bei dem erfindungsgemäßen Verfahren alle üblicherweise für derartige Umsetzungen verwendbaren Säurebindemittel eingesetzt werden. Vorzugsweise in Frage kommen alkalicarbonate wie Natrium- und Kaliumcarbonat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0°C und 100°C, vorzugsweise bei Temperaturen zwischen 40°C und 80°C. Das erfindungsgemäße Verfahren wird im allgemeinen unter Normaldruck durchgeführt.

Zur bevorzugten Durchführung des erfindungsgemäßen Verfahrens leitet man durch ein Gemisch aus Ausgangsprodukt der Formel (II), Säureakzeptor und Verdünnungsmittel elementares Chlor ein und das Reaktionsgemisch wird mehrere Stunden bei der jeweils erforderlichen Temperatur (vorzugsweise im Bereich von 40 bis 80°C) gerührt. Die Aufarbeitung erfolgt nach allgemein üblichen Methoden.

Das nach dem erfindungsgemäßen Verfahren herzustellende 2-Chlor-5-chlormethylpyridin kann beispielsweise als Zwischenprodukt für die Herstellung von Nitromethylen-Derivaten, die als Insektizide wirksam sind, eingesetzt werden (vgl. EP-A 163 855).

In diesem Zusammenhang sei beispielhaft das folgende Weiterverarbeitungsschema aufgeführt:

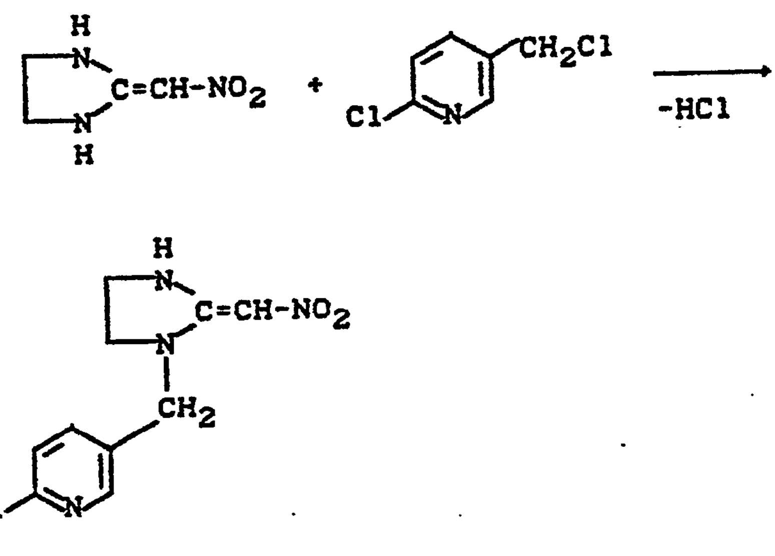


Herstellungsbeispiel

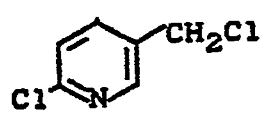

Durch eine Lösung von 2,54 g (0,02 Mol) 2-Chlor-5-methylpyridin und 4 g (0,0265 Mol) Nariumcarbonat in 10 ml Tetrachlorkohlenstoff wird bei 60°C elementares Chlor eingeleitet. Der Reaktionsvorgang wird gaschromatographisch verfolgt. Nach 10 Stunden wird das Reaktionsgemisch abgekühlt und eingeengt.

Man erhält 2,1 g (65% der Theorie) 2-Chlor-5-chlormethylpyridin. Die Struktur wird durch 1H-NMR-Spektren gesichert.

1H-NMR ($CDCl_3$) : δ = 8,4 (d, 1H, –CH–N=), 7,73 (dd, 1H,

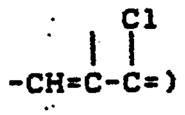, 7,35 (d, 1H, 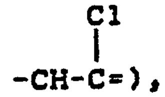,

4,57 (s, 2H, $-CH_2$) ppm.

,

**Patentansprüche**

1. Verfahren zur Herstellung von 2-Chlor-5-chlormethyl-pyridin der Formel (I)

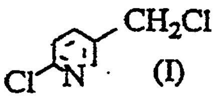

(I)

dadurch gekennzeichnet, daß man 2-Chlor-5-methyl-pyridin der Formel (II)

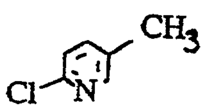

gegebenenfalls in Gegenwart von Säureakzeptoren und gegebenenfalls in Gegenwart von inerten Verdünnungsmitteln bei Temperaturen zwischen 0°C und 100°C mit elementarem Chlor chloriert.

2. Verfahren gemäß Anspruch 1, dadurch gekennzeichnet, daß die Umsetzung in Gegenwart von Säureakzeptoren und in Gegenwart von inerten Verdünnungsmitteln durchgeführt wird.

3. Verfahren gemäß Anspruch 1 und 2, dadurch gekennzeichnet, daß als inerte Verdünnungsmittel aliphatische, gegebenenfalls halogenierte Kohlenwasserstoffe oder Ether eingesetzt werden.

4. Verfahren gemäß Anspruch 1 bis 3, dadurch gekennzeichnet, daß als Säureakzeptoren Alkalicarbonate, aliphatische, aromatische oder heterocyclische Amine eingesetzt werden.

5. Verfahren gemäß Anspruch 1 bis 4, dadurch gekennzeichnet, daß die Umsetzung bei Temperaturen zwischen 40 und 80°C durchgeführt wird.

6. Verfahren gemäß Anspruch 1 bis 5, dadurch gekennzeichnet, daß man durch ein Gemisch aus Ausgangsprodukt der Formel (II), Säureakzeptor und Verdünnungsmittel, elementares Chlor einleitet und das Reaktionsgemisch mehrere Stunden gerührt wird.

**Claims**

1. Process for the preparation of 2-chloro-5-chloromethyl-pyridines of the formula (I)

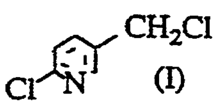

(I)

characterized in that 2-chloro-5-methylpyridines of the formula (II)

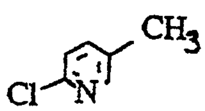

are chlorinated with elemental chlorine at temperatures between 0°C and 100°C, if appropriate in the presence of inert diluents.

2. Process according to Claim 1, characterized in that the reaction is carried out in the presence of acid acceptors and in the presence of inert diluents.

3. Process according to Claims 1 and 2, characterized in that the inert diluents employed are aliphatic, optionally halogenated hydrocarbons, or ethers.

4. Process according to Claims 1 to 3, characterized in that the acid acceptors employed are alkali

metal carbonates, or aliphatic, aromatic or heterocyclic amines.

5. Process according to Claims 1 to 4, characterized in that the reaction is carried out at temperatures between 40 and 80°C.

6. Process according to Claim 1 to 5, characterized in that elemental chlorine is passed through a mixture of the starting material of the formula (II), acid acceptor and diluent, and the reaction mixture is stirred for several hours.

**Revendications**

1. Procédé pour la fabrication de chloro-2-chlorométhyl-5-pyridine de formule (I)

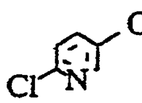

(I)

caractérisé en ce que l'on chlore la chloro-2-méthyl-5-pyridine de formule (II)

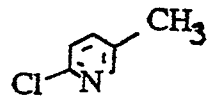

par le chlore élémentaire à des températures entre 0 et 100°C, éventuellement en présence d'accepteurs d'acides et éventuellement en présence de diluants inertes.

2. Procédé selon la revendication 1, caractérisé en ce que la rèaction est mise en œuvre en présence d'accepteurs d'acides et de diluants inertes.

3. Procédé selon les revendications 1 et 2, caractérisé en ce que l'on utilise comme diluants inertes des hydrocarbures aliphatiques, éventuellement halogénés ou des éthers.

4. Procédé selon les revendications 1 à 3, caractérisé en ce que l'on utilise comme accepteurs d'acides des carbonates alcalins ou des amines aliphatiques, aromatiques ou hétérocycliques.

5. Procédé selon les revendications 1 à 4, caractérisé en ce que la réaction est mise en œuvre à des températures entre 40 et 80°C.

6. Procédé selon les revendications 1 à 5, caractérisé en ce que l'on envoie du chlore élémentaire dans un mélange du produit de départ de formule (II), de l'accepteur d'acide et du diluant et on agite le mélange de réaction pendant plusieurs heures.